# EUROPÄISCHE PATENTSCHRIFT

(11) **EP 2 359 896 B1**
(45) Veröffentlichungstag und Bekanntmachung des Hinweises auf die Patenterteilung: **27.01.2016**
(21) Anmeldenummer: 11153009.3
(22) Anmeldetag: 02.02.2011
(51) Int. Cl.: A61N 1/05, A61N 1/08

(54) **Elektrodenvorrichtung zur Strom- oder Spannungsführung zwischen einem implantierbaren elektromedizinischen Gerät und einem Therapie- und/oder Diagnoseort im menschlichen Körper**
Electrode Unit for Carrying Current or Voltage between an Implantable Electromedical Device and a Treatment and/or Diagnosis Site in the Human Body
Dispositif d'électrodes pour la commande de courant ou de tension entre un appareil électromédical implantable et un lieu de thérapie et/ou diagnostic dans le corps humain

(30) Priorität: 11.02.2010 DE 102010000373
(43) Veröffentlichungstag der Anmeldung: 24.08.2011
(73) Patentinhaber: BIOTRONIK SE & Co. KG, 12359 Berlin (DE)
(72) Erfinder: Weiss, Ingo, 12435, Berlin (DE); Friedrich, Michael, 14532, Kleinmachnow (DE); Knorr, Stefan, 10119, Berlin (DE); Fischer, René, 10247, Berlin (DE); Schurr, Marc Steffen, 10179, Berlin (DE)
(74) Vertreter: Galander, Marcus

(56) Entgegenhaltungen:
- EP-A1- 2 025 361
- US-A1- 2008 058 902
- US-A1- 2008 058 913

## Beschreibung

Die Erfindung betrifft eine Elektrodenvorrichtung zur Strom- oder Spannungsführung zwischen einem implantierbaren elektromedizinischen Gerät und einem Therapie- und/oder Diagnoseort im menschlichen Körper, wobei die Elektrodenvorrichtung mindestens eine strom-/spannungsführende Zuleitung und mindestens einen elektrischen Kontaktpol zu einem Körperteil aufweist.

Zum Hindergrund der Erfindung ist festzuhalten, dass der Erfindungsgegenstand in erster Linie im Zusammenhang mit Herzschrittmachern, implantierbaren Defibrillatoren und anderen Typen von aktiven implantierbaren elektromedizinischen Geräten relevant ist. Letztere weisen in aller Regel mindestens eine strom-/spannungsführende Zuleitung in der Elektrodenvorrichtung - üblicherweise als kurz als "Elektrode" bezeichnet - auf, deren distales Ende beispielsweise in einer Herzkammer angeordnet und zur Messung kardiologischer Potentialsignale oder zur Abgabe entsprechender therapeutischer Stromsignale dient.

Die Kompatibilität derartiger Elektrodenvorrichtungen bei implantierbaren elektromedizinischen Geräten mit hochfrequenten Magnetfeldern, wie sie insbesondere bei bildgebenden diagnostischen Verfahren auf Magnetresonanz-Basis - sogenannte MRI- (magnetic resonance imaging) Verfahren - verwendet werden, stellt ein gravierendes Problem dar. Bei solchen MRI-Verfahren wird einem starken statischen Magnetfeld ein mit Radiofrequenz (RF) gepulstes magnetisches Wechselfeld überlagert, das dazu dient, den Energiestatus der Protonen im untersuchten Gewebe zu verändern und entsprechende MRI-Signale aus dem Gewebe zu produzieren.

Dieses magnetische Wechselfeld führt nach den Gesetzen der elektromagnetischen Induktion in den Zuleitung der hier in Rede stehenden Elektrodenvorrichtungen von elektromedizinischen Geräteimplantaten zu Wechselspannungen, deren Energie insbesondere an den elektrisch leitenden Kontaktpolen der Elektrodenvorrichtung zum menschlichen Gewebe in Wärme umgesetzt wird. Dies kann zu einer erheblichen Erhitzung beispielsweise des Spitzenkontaktes einer Herzelektrode mit einer entsprechenden Beeinträchtigung und sogar Schädigung des damit in Berührung stehenden oder umliegenden Herzgewebes führen. Um diese Problematik zu vermeiden, schlägt die US 7,363,090 B2 die Verwendung von Filtern auf der Basis von Schwingkreisen aus parallel geschalteter Spule und Kondensator vor, die der entsprechenden Zuleitung für den Spitzen-Kontaktpol oder einen Ring-Kontaktpol einer entsprechenden Elektrode eines implantierbaren elektromedizinischen Gerätes zugeordnet ist. Die in diesem vorbekannten Patent offenbarten Filter sind in der Praxisumsetzung der Patentinhaberin als vergleichsweise voluminöse, die Elektrodenvorrichtung auf einer gewissen Länge versteifende Komponenten ausgebildet, die der damit ausgerüsteten Elektrode ungünstige mechanische Eigenschaften verleihen. Ferner ist der Filter in einem in sich geschlossenen Gehäuse untergebracht, das keine Durchführung für die in aller Regel beim Implantieren einer Elektrode verwendeten Führungsdrähte bereit stellt. Insoweit sind die Einsatzmöglichkeiten dieser bekannten Elektrode mit Filtereinrichtung begrenzt.

Eine Elektrodenvorrichtung gemäß ersten Teil von Anspruch 1 ist aus US 2008/0058913 A1 bekannt. Dort wird vorgeschlagen, die wendelförmige frequenzabhängige Übertragungseinrichtung zur Herstellung gewünschter elektrischer Induktivitäten zu beschichten. Hierzu werden isolierende Materialien, oder Materialien mit endlichen elektrischen Widerstand, oder Materialien mit Ferritanteilen vorgeschlagen.

Der Erfindung liegt die Aufgabe zugrunde alternative Ausführungsmöglichkeiten aufzuzeigen.

Diese Aufgabe wird in ihrer allgemeinsten Ausprägung durch die kennzeichnenden Merkmale des Anspruches 1 gelöst. Demnach ist der strom-/spannungsführenden Zuleitung der Elektrodenvorrichtung mindestens ein Abschnitt mit einem frequenzabhängigen Übertragungsverhalten zugeordnet, wobei dieser mindestens eine frequenzabhängige Übertragungsabschnitt durch einen höher leitfähigen Abschnitt einer Wendel der strom-/spannungsführenden Zuleitung gebildet ist.

Durch dieses frequenzabhängige Übertragungsverhalten ist die Elektrodenvorrichtung so angepasst, dass sie in einem Magnetresonanz-Umfeld schädliche Wechselspannungen aufgrund des magnetischen Wechselfeldes ausfiltert und eine Erwärmung der Zuleitung zu dem elektrischen Kontaktpol der Elektrodenvorrichtung wirkungsvoll unterbindet. Insoweit fließen also keine unerwünschten Ströme in den Körper des Patienten. Die erfindungsgemäß vorgesehene Ausfilterung von Frequenzen oder Frequenzbereichen kann sich auch in einer Umverteilung der induzierten Wechselströme in der Zuleitung zwischen verschiedenen Kontaktpolen äußern, was ebenfalls eine Erwärmung der Zuleitung und der Kontaktpole wirkungsvoll verhindert.

Gemäß bevorzugter Weiterbildungen der Erfindung kann das frequenzabhängige Übertragungsverhalten einem Tiefpass-, Bandsperr-, Bandpass- oder Hochpassfilter entsprechen. Die Tiefpass-Charakteristik kann beispielsweise eine Grenzfrequenz von 100 kHz, besonders bevorzugt 10kHz, mit einer Flanke von > = 6 dB/Oktave aufweisen. Bei einem Bandsperrfilter kann das Frequenz-Sperrband beispielsweise zwischen 10 MHz und 3 Ghz liegen. Diese Frequenzcharakteristik ist für das frequenzabhängige Übertragungsverhalten vom implantierten elektromedizinischen Gerät zum Therapie-/Diagnoseort genauso von Vorteil wie das oben erwähnte Tiefpass-Verhalten.

Für das frequenzabhängige Übertragungsverhalten zwischen verschiedenen Kontaktpolen am Therapie-/Diagnoseort ist die erwähnte Hochpass-Charakteristik mit einer Grenzfrequenz > 100 kHz und einer Flanke von > 6 dB/Oktave oder ein Bandpassverhalten mit einer Mittenfrequenz zwischen 10 MHz und 3 Ghz von Vorteil. Die Filtergüte bei einem Bandsperr- oder Hochpass-Filter sollte einen Wert von über 20 aufweisen.

Zu dem die Strom-/Spannungsführung bildenden Leiter ist festzuhalten, dass dieser eine Isolation aufweist, die eine mindestens 100-fach geringere Gleichstrom-Leitfähigkeit als der Leiter selbst aufweist. Letzterer kann üblicher Weise aus Metall, aber auch aus leitendem Kunststoff, Kohlefasern, einer leitfähigen Flüssigkeit usw. bestehen.

Gemäß einer bevorzugten Ausführungsform der Erfindung kann die frequenzabhängige Übertragungseinrichtung eine Durchführung mit einem vorzugsweisen Mindestdurchmesser von 0,2 mm für einen Führungsdraht, Guidewire, Mandrin oder dergleichen aufweisen. Im Gegensatz zum Stand der Technik ist damit die erfindungsgemäße Elektrodenvorrichtung trotz der vorhandenen Ausfilterung induzierter Spannungen über einen Führungsdraht in entsprechende Körpergefäße einführbar.

Grundsätzlich kann die frequenzabhängige Übertragungseinrichtung eine oder mehrere Zuleitungen in der implantierbaren Elektrodenvorrichtung kontaktieren. Die Übertragungseinrichtung ist dann an geeigneten Positionen der Zuleitung daran angeschlossen.

Erfindungsgemäß ist die frequenzabhängige Übertragungseinrichtung durch einen oder mehrere Abschnitte der strom-/spannungsführenden Zuleitung selbst gebildet, die durch einen höher leitfähigen Abschnitt einer Wendel der Zuleitung ausgeführt sind. Bevorzugtermaßen sollte ein entsprechender Abschnitt der gewendelten Zuleitung eine mindest doppelt so hohe elektrische Leitfähigkeit wie die umgebenden Bereiche der Zuleitung aufweisen. Die höher leitfähige Auslegung des entsprechenden Wendelabschnittes kann durch eine hoch leitfähige Beschichtung oder eine Dotierung des Wendelmaterials in diesem Abschnitt hervorgerufen werden. Dabei sollten vorzugsweise die Windungen in dem höher leitfähigen Abschnitt der Wendel nach außen isoliert sein, damit zwischen den einzelnen Windungen der Wendel keine Kurzschlüsse auftreten.

Eine konstruktiv vorteilhafte Ausbildung einer entsprechenden frequenzabhängigen Übertragungseinrichtung ist durch einen Induktivität-Kapazität-(LC)-Resonator realisiert, bei dem der höher leitfähige Abschnitt der Wendel als Induktivität mit einem parallel geschalteten Kondensator kombiniert ist. Bevorzugtermaßen ist dabei der höher leitfähige Wendelabschnitt, der die Induktivität realisiert, beispielsweise vergoldet, um eine hohe Güte des LC-Schwingkreises zur gewährleisten.

Eine sehr kompakte Weiterbildung eines solchen LC-Resonators ergibt sich durch eine Erfindungsvariante, bei der der Kondensator durch einen um den leitfähigen Abschnitt der Wendel herum und/oder innerhalb des leitfähigen Abschnitts der Wendel angeordneten Zylinder-Kondensator gebildet ist. Dies stellt eine besonders klein bauende Ausführungsform der erfindungsgemäßen Elektrodenvorrichtung dar, bei der der Wendelinnenraum als Durchführung für einen Führungsdraht zur Verfügung bleibt und/oder gerade dieser für den Aufbau genutzt wird.

Gemäß einer weiteren bevorzugten Weiterbildung der Erfindung ist die Frequenzabhängigkeit der Übertragungseinrichtung variabel steuerbar. Damit kann die Elektrodenvorrichtung an die jeweiligen Umstände, nämlich die Frequenz der induzierten Störsignale angepasst werden. Damit bleibt die Elektrodenvorrichtung unabhängig von ihrer MRI-Umgebung universell einsetzbar.

Bevorzugter Weise erfolgt die Steuerung der Frequenzabhängigkeit mit Hilfe eines Trimmelementes, wie etwa einem spannungsgesteuerten Kondensator, einem sogenannten Varicap oder einem Varactor. Auch eine Biasspannung ist zur Frequenzsteuerung verwendbar. Eine solche Steuerspannung kann durch eine Programmierung des elektromedizinischen Gerätes selbst oder durch einen Sensor zur Erfassung therapeutisch und/oder diagnostisch unerwünschter Signale erzeugt werden. Der Sensor kann dabei eine Störfrequenz in einem vorgegebenen Frequenzband und/oder mit einer bestimmten Amplitude größer einer einstellbaren Schwelle detektieren. Insoweit kann eine Elektrodenvorrichtung eines implantierbaren elektromedizinischen Gerätes in mehreren üblichen Magnetfeldbereichen von MRI-Systemen, also beispielsweise 1,5 und 3,0 T im Gegensatz zu einer konstruktiv fest verdrahteten Variante eingesetzt werden.

Für die Positionierung des Sensors bestehen verschiedene bevorzugte Varianten, so kann er im implantierten elektromedizinischen Gerät selbst angeordnet oder in der Zuleitung der Elektrodenvorrichtung zugeordnet sein. Im erstgenannten Fall führen entsprechende Steuerleitungen zur frequenzabhängigen Übertragungseinrichtung, um deren Frequenzabhängigkeit zu steuern.

Bei einer Zuordnung des Sensors zur Zuleitung arbeitet der Sensor im Wesentlichen autark, also ohne das implantierbare elektromedizinische Gerät, womit sich der Verdrahtungsaufwand für die Steuerung der Frequenzabhängigkeit verringert. Als Sensor kommt vorteilhafter Weise ein Feldsensor für elektrische, magnetische und/oder elektromagnetische Felder, wobei die Felder statische und/oder Wechselfelder sind, in Form insbesondere eines Dipols zum Einsatz.

Eine weitere Variante für die frequenzabhängige Übertragungseinrichtung liegt in deren Bildung durch einen Schwingkreis. Dabei wird das frequenzabhängige Übertragungsverhalten durch elektrische Wirkelemente in Form eines Widerstands R, einer Kapazität C, Induktivität L oder mit Hilfe von Überträgern Ü bedarfsgerecht realisiert. Dabei kommen Wirkelemente-Kombinationen wie RL, RC, RLC, RÜ, CÜ oder RLCÜ zum Einsatz. Ein oder mehrere solche Schwingkreise können kombiniert werden, wobei vorzugsweise eine induktive Ankopplung an eine oder mehrere Zuleitungen möglich ist.

Eine konstruktiv geschickte Anbindung der frequenzabhängigen Übertragungseinrichtung ist möglich, indem diese an einer Fixierhelix an der Elektrodenvorrichtung ausgebildet ist. Insbesondere ist eine Induktivität aus einem Teil der Fixierhelix geformt.

Eine weitere konzeptionelle Variante für die frequenzabhängige Übertragungseinrichtung liegt in der Realisierung mit Hilfe eines oder mehrerer Wellenleiter, die eine frequenzspezifische Wellenimpedanz aufweisen. Das Wellenleiterkonzept kann dabei für sich oder auch in Kombination mit den anderen Frequenzsteuerungsmechanismen, wie den oben erwähnten Schwingkreisen kombiniert werden.

Bevorzugter Weise sind die oder der Wellenleiter durch eine Abschlussimpedanz abgeschlossen, bei der es sich im einfachsten Fall um einen Kurzschluss handelt.

Zur Frequenzanpassung an mindestens zwei unerwünschte Signalfrequenzen können in den oder die Wellenleiter Netzwerke zwischengeschaltet sein und/oder der Abschluss eines Wellenleiters durch ein Netzwerk erfolgen. Damit ist eine hohe Variabilität in der Frequenzanpassung erreichbar.

Grundsätzlich kann jeder Wellenleiter durch zwei über ein Dielektrikum gekoppelte Leiter gebildet sein.

Eine in sich kompakte Ausführung für die Zuordnung des Wellenleiters zu einer strom-/spannungsführenden Zuleitung ist durch eine gewendelte Ausbildung des Wellenleiters und eine Integration in eine als Wendelleitung ausgebildete strom-/spannungsführende Zuleitung gegeben.

Weitere Merkmale, Einzelheiten und Vorteile der Erfindung ergeben sich aus den nachfolgenden Beschreibungen einer Vielzahl von Ausführungsbeispielen anhand der beigefügten Zeichnungen. Es zeigen:
- Fig. 1: eine schematische Ansicht einer Zuleitung einer Elektrodenvorrichtung zur Strom- oder Spannungsführung zwischen einem implantierbaren elektromedizinischen Gerät und einem Therapie- und/oder Diagnoseort mit einer integrierten frequenzabhängigen Übertragungseinrichtung und einen Detail-Querschnitt durch die Zuleitung,
- Fig. 2a und b: Ersatzschaltbilder für die frequenzabhängige Übertragungseinrichtung in Form eines LC-Gliedes mit fester und veränderlicher Kapazität des Kondensators
- Fig. 3: eine schematische Darstellung einer Zuleitung mit integrierter frequenzabhängiger Übertragungseinrichtung in einer weiteren Ausführungsform,
- Fig. 4: ein Blockdiagramm einer Zuleitung mit Übertragungseinrichtung mit einer steuerbaren Frequenzabhängigkeit,
- Fig. 5: ein Schaltbild einer schaltungstechnischen Realisierung der Anordnung gemäß Fig. 4,
- Fig. 6: eine schematische Ansicht einer in eine Fixierhelix einer Elektrodenvorrichtung integrierten frequenzabhängigen Übertragungseinrichtung,
- Fig. 7: ein Schaltbild einer Elektrodenvorrichtung mit zwei Zuleitungen und Schwingkreisen als frequenzabhängige Übertragungseinrichtungen,
- Fig. 8: das Dämpfungsverhalten einer frequenzabhängigen Übertragungseinrichtung in Form eines elliptischen Tiefpasses 8. Ordnung,
- Fig. 9: einen Schaltplan einer Elektrodenvorrichtung mit zwei Zuleitungen und induktiv angekoppelten Schwingkreisen als frequenzabhängige Übertragungseinrichtung,
- Fig. 10: eine Variante der Elektrodenvorrichtung von Fig. 7 mit in beide Zuleitungen integrierten Schwingkreisen,
- Fig. 11: ein Schaltbild einer Elektrodenvorrichtung mit zwei Zuleitungen, die induktive Abschnitte und kapazitive Kreuzkopplungen aufweisen,
- Fig. 12 und 13: analoge Schaltpläne von Elektrodenvorrichtungen mit drei Zuleitungen,
- Fig. 14: eine schematische Ansicht einer Elektrodenvorrichtung mit zwei Zuleitungen und einem Wellenleiter als frequenzabhängige Übertragungseinrichtung,
- Fig. 15 bis 17: schematische ausschnittsweise Perspektivdarstellungen eines Wellenleiters in unterschiedlichen Realisierungen,
- Fig. 18: eine schematische Ansicht einer Elektrodenvorrichtung mit zwei Kontaktpolen und Zuleitungen und Wellenleitern als frequenzabhängige Übertragungseinrichtungen,
- Fig. 19: eine Elektrodenvorrichtung analog Fig. 18 mit zusätzlichen Netzwerken in den Wellenleitern,
- Fig. 20: einen schematischen Detailausschnitt eines Wellenleiters mit Netzwerk gemäß Fig. 19,
- Fig. 21A: bis l Schaltschemata unterschiedlicher Varianten für die Netzwerke gemäß Fig. 19 und 20,
- Fig. 22: eine weitere alternative Ausführungsform eines in eine Zuleitung integrierten Wellenleiters als frequenzabhängige Übertragungseinrichtung,
- Fig. 23: eine schematische Darstellung einer Elektrodenvorrichtung mit Innen- und Außenleiter und diesen zugeordneten frequenzabhängigen Übertragungseinrichtungen,
- Fig. 24A/B - Fig. 26A/B: schematische Darstellungen von Elektrodenvorrichtungen mit integrierten frequenzabhängigen Übertragungseinrichtungen mit den entsprechenden Ersatzschaltbildern.

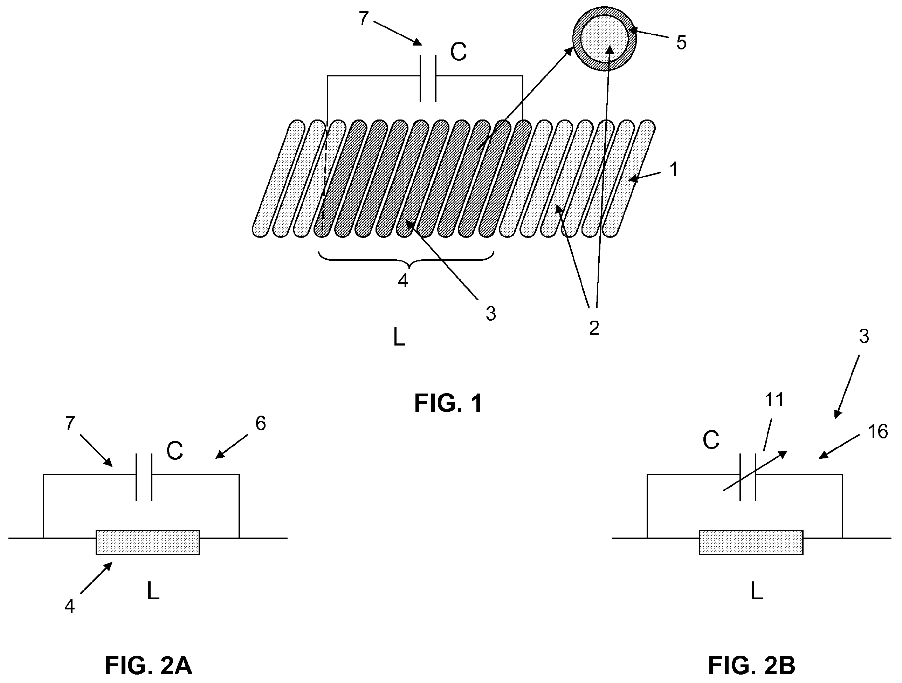

Die Fig. 1 zeigt im Zusammenhang mit einer nicht insgesamt dargestellten Elektrodenvorrichtung zur Strom- oder Spannungsführung zwischen einem implantierbaren elektromedizinischen Gerät und einem Therapie- und/oder Diagnoseort, wie sie beispielsweise durch eine Herzschrittmacher-Elektrode repräsentiert ist, im wesentlichen lediglich eine Zuleitung 1, die in Form einer Wendel 2 aus einem üblichen medizinischen Stahl, wie MP35N besteht. Die Zuleitung 1 führt zu einem nicht gezeigten elektrischen Kontaktpol, der mit einem Körperteil in elektrischem Kontakt steht und der beispielsweise durch einen Tip- oder Ring-Kontaktpol der Herzschrittmacherelektrode repräsentiert ist.

Das grundsätzliche Konzept der vorliegenden Erfindung erschließt sich aus dieser Fig. 1 dahingehend, dass eine Einrichtung 3 mit einem frequenzabhängigen Übertragungsverhalten vorgesehen ist, die therapeutisch und/oder diagnostisch unerwünschte Signale in mindestens einem definierten Frequenzbereich, wie beispielsweise über 10 MHz zumindest teilweise ausfiltert und damit die eingangs beschriebenen Probleme bei der Beaufschlagung der Elektrodenvorrichtung mit einem hochfrequenten magnetischen Wechselfeld vermeidet.

Im gezeigten Ausführungsbeispiel gemäß Fig. 1 ist die frequenzabhängige Übertragungseinrichtung 3 durch einen LC-Resonanzkreis gebildet, wobei die Induktivität L durch einen sich über mehrere Windungen der Wendel 2 erstreckenden Abschnitt 4 gebildet ist, der aus einem deutlich, mindestens zweifach besser elektrisch leitfähigen Material wie die restlichen Wendeln besteht. Die höhere Leitfähigkeit wird - wie aus dem in Fig. 1 integrierten Detailquerschnitt deutlich wird - durch eine hochleitende Beschichtung 5 beispielsweise in Form einer Vergoldung hervorgerufen. Damit ist eine hohe Güte des durch die Induktivität L und die parallel dazu geschaltete Kapazität C gebildeten Schwingkreises 6 gewährleistet.

Fig. 2 zeigt das Ersatzschaltbild der frequenzabhängigen Übertragungseinrichtung 3 in Form eines LC-Schwingkreises 6.

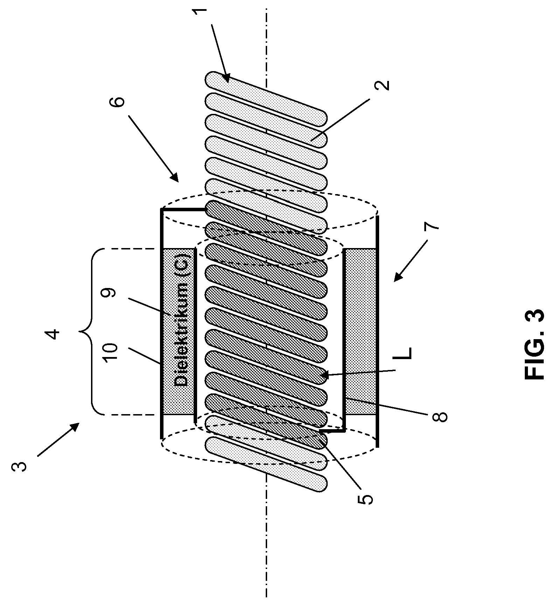

In Fig. 3 ist schematisch eine Realisierung des Schwingkreises 6 mit der beschriebenen Induktivität L und einem um den entsprechenden Abschnitt 4 der Wendel 2 herum angeordneten Kondensator 7 gezeigt. Letzterer ist als Zylinderkondensator mit einer Innenhülse 8 und einer über ein Dielektrikum 9 getrennten Außenhülse 10 realisiert. Die beiden Hülsen 8, 10 sind dabei jeweils mit den Wendelwindungen an den entgegengesetzten Enden des Abschnittes 4 elektrisch verbunden, wodurch die in Fig. 2a gezeigte Parallelschaltung der Induktivität L und der Kapazität C hervorgerufen wird. Das Dielektrikum 9 hat einen Dielektrizitätswert von eps_r >=2.5. Unterhalb von eps_r = 2.5 werden Bauelement zu groß. Im Bereich eps_r = 2.5 bis 10 deckt man dann die gängigsten Kunststoffe, auch Glas, ab. Bevorzugt sind eps_r >=10 bis 100, um die Baugröße gering zu halten. Dieser Bereich beinhaltet zum Beispiel auch Al203 oder Ta2O5. In einer besonderen Ausführung ist eine Hülse mit dem darauf gewachsenen Oxid, das damit gleich auch das Dielektrikum bildet (Al203 oder Ta2O5 oder Oxide anderer Ventilmetalle)
Weiter bevorzugt wären dann Keramiken als Dielektrikum mit eps_r > 100 bis einige tausend z.B. Bariumtitanat.

Wie in Fig. 2b angedeutet ist, kann die Frequenzabhängigkeit der frequenzabhängigen Übertragungseinrichtung 3 auch variabel steuerbar sein, indem beispielsweise die Kapazität C variabel ist. Die Trimmung des kapazitiven Elementes kann von dem (nicht dargestellten) Implantat, also beispielsweise einem Herzschrittmacher oder Defibrillator durch eine Bias-Spannung vorgenommen werden. Das entsprechende Trimmelement 11 kann beispielsweise ein spannungsgesteuerter Kondensator, Varicap oder Varactor sein. Die Steuerparameter sind dabei in das Implantat durch externe Programmierung eingebbar.

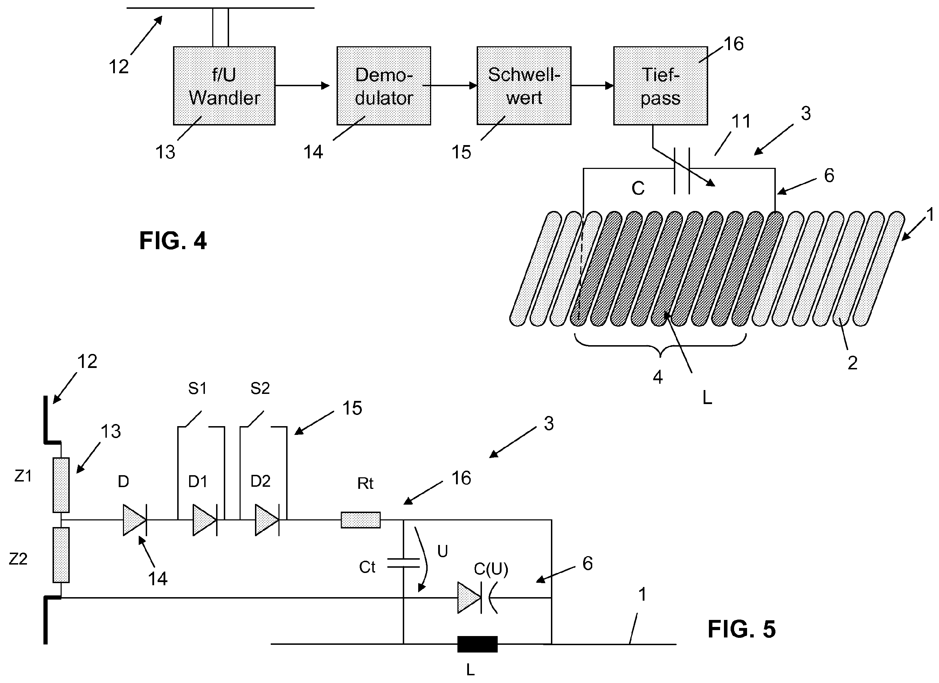

Alternativ dazu kann ein Sensor 12, insbesondere ein Feldsensor für das die Elektrodenvorrichtung beaufschlagende statische magnetische Feld, also die Stärke des Magnetfeldes, und/oder die Frequenz eines HF Feldes vorgesehen sein, der therapeutisch und/oder diagnostisch unerwünschte Signale erfasst. Eine entsprechende Elektrodenvorrichtung ist in den Fig. 4 und 5 dargestellt. Dem Sensor 12 in Form eines Dipols sind ein f/U-Wandler (Frequenz-Spannungs Wandler) 13, ein Demodulator 14, ein Schwellwertgeber 15 und ein Tiefpass 16 nachgeschaltet. Über letzteren ist das Trimmelement 11 einstellbar. Insgesamt erfasst die in Fig. 4 gezeigte Beschaltung des Trimmelementes 11 eine Störfrequenz in einem vorgegebenen Band und mit einer Amplitude größer einer einstellbaren Schwelle. Die entsprechenden Parameter orientieren sich an üblichen Magnetfeldern, wie sie bei bekannten MRI-Systemen mit einer Magnetfeldstärke von 1,5 T und 3 T eingesetzt werden. Der Sensor 12 kann im Implantat angeordnet sein, wobei Steuerleitungen zur frequenzabhängigen Übertragungseinrichtung 3 führen, zu bevorzugen ist jedoch die in Fig. 4 und 5 dargestellte autarke Auslegung, bei der der Sensor 12 im Bereich der Elektrodenvorrichtung selbst montiert ist.

Fig. 5 zeigt eine konkrete Implementierung der in Fig. 4 diagrammartig dargestellte Schaltung. So ist der Sensor 12 ein Dipol, wobei der Frequenz/Spannungs-(f/U)Wandler 13 durch einen Spannungsteiler aus zwei Impedanzen Z1 und Z2 realisiert ist. Alternativ dazu können abgestimmte Resonatoren für bestimmte Frequenzen die entsprechende Vorspannung steuern. Im gezeigten Beispiel wäre dann Z1 ein Widerstand und Z2 eine Induktivität.

Der Demodulator 14 ist durch eine Diode D repräsentiert, der ein Schwellwertgeber 15 in Form der durch Schalter S1, S2 überbrückbaren Dioden D1, D2 folgt. Der Tiefpassfilter 16 ist als RC-Schaltung mit dem Widerstand Rt und dem Kondensator Ct realisiert.

Die so geglättete, dem anliegenden Magnetfeld entsprechende Gleichspannung steuert dann die spannungsabhängige Kapazität C(U) des Schwingkreises 6 an. Dessen Induktivität L liegt in der Elektroden-Zuleitung 1.

Die spannungsabhängige Kapazität C(U) kann eine Kapazitätsdiode (Abstimmdiode, Varicap, Varactor) sein oder wie in der EP 1 299 948 B1 realisiert sein. Da bei einer Auslegung der Impedanz Z1 als Widerstand und der Impedanz Z2 als Induktivität die Gleichspannung U mit der Frequenz f ansteigt, muss die Kapazität C(U) so realisiert sein, dass die Kapazität C mit steigender Steuerspannung U kleiner wird, damit die Wirkfrequenz der Sperrvorrichtung erhöht wird. Um umgekehrten Fall müsste die Impedanz Z2 als Widerstand und die Impedanz Z1 als Induktivität gewählt werden.

Für die Variante einer Bandsperre ist zu beachten, dass die Kennlinie der spannungsabhängigen Kapazität C(U) so realisiert wird, dass die Wirkfrequenz der mit dem Feldsensor 12 erfassten Frequenz f folgt. Alternativ kann auch der Frequenz-/Spannungswandler 13 mit einer entsprechend kompensativen Kennlinie ausgebildet sein.

Die Möglichkeit einer derartigen Justierung der Kapazität gilt im Übrigen für alle in dieser Anmeldung beschriebenen Kapazitäten.

Die vorstehend erörterten Varianten der Erfindung sind nochmals kurz wie folgt zu umreißen:
- Der Sensor 12 ist Teil eines Reglers, der das frequenzabhängige Übertragungsverhalten an die Frequenz des gerade vorliegenden Störsignals anpasst.
- Die Übertragungseinrichtung 3 mit frequenzabhängigem Übertragungsverhalten ist mit elektrischen Wirkelementen realisierbar, wie eine RL-, RC-, RLC-, RÜ-, CÜ-, RLCÜ-Schaltung, wobei Ü "Übertrager" bedeutet.
- Wie später noch erläutert wird, können für die frequenzabhängige Übertragung auch Wellenleiter herangezogen werden, die gegebenenfalls auch in Kombination mit den vorstehend erörterten Schwingkreis-Schaltungen verwendbar sind.
- Die elektrischen Wirkelemente sind durch verteilte Parameter realisiert (Materialeigenschaften, z.B. ein Werkstoff der mechanisch ein Stück ist aber dessen Materialeigenschaften örtlich so verteilt sind, dass sich funktional bestimmte elektrische Bauelemente ausbilden: Z.B. Sandwich aus: leitender Werkstoff, nichtleitenden (besonders dielektrisch ausgeprägter) Werkstoff, leitender Werkstoff bilden in der Flussrichtung dieser Aufzählung einen Kondensator)
- Alle vorstehenden Maßnahmen können kombiniert werden.
- Die Dämpfung, die durch eine oder als Gesamteffekt mehrerer frequenzabhängiger Übertragungseinrichtungen für die Störsignale realisierbar ist, kann über 10 dB betragen.
- Das frequenzabhängige Übertragungsverhalten (Tiefpass, Hochpass, Bandpass, Bandsperre) wird durch Einsatz von Schwingkreisen oder Wellenleitern realisiert.
- Die induktiven Elemente (L, Ü) der frequenzabhängigen Übertragungseinrichtungen werden durch Ausnutzung vorhandener induktiver Abschnitte der Zuleitung 1 realisiert, beispielsweise in Wendeln von Schrittmacherelektroden. Einfache Seilleitungen können für diesen Zweck auch lokal gewendelt werden.
- Zur Erhöhung der Güte des Schwingkreises und damit zur Erhöhung der Dämpfung der frequenzabhängigen Übertragungseinrichtungen werden Leiter mit hoher Leitfähigkeit, also beispielweise Kupfer-, Gold- oder Silberdrähte verwendet. Bei Einsatz vorhandener induktiver Abschnitte der Zuleitung werden deren Ohm'scher Widerstand und damit die Verlustleistung durch Aufbringen von hochleitfähigen Schichten auf diesen Abschnitt verringert. Dies kann durch Galvanisieren oder Bedampfen erfolgen.
- Die mechanische Verbindung der elektrischen Wirkelemente ist durch übliche Maßnahmen, wie feste Verbindungen in Form von Schweißen, Löten oder Crimpen zu erzielen. Mobile elektrische Verbindungen können durch Schleifkontakte, biegbare Zwischenelemente oder Flüssig-Metall-Übergänge erreicht werden.

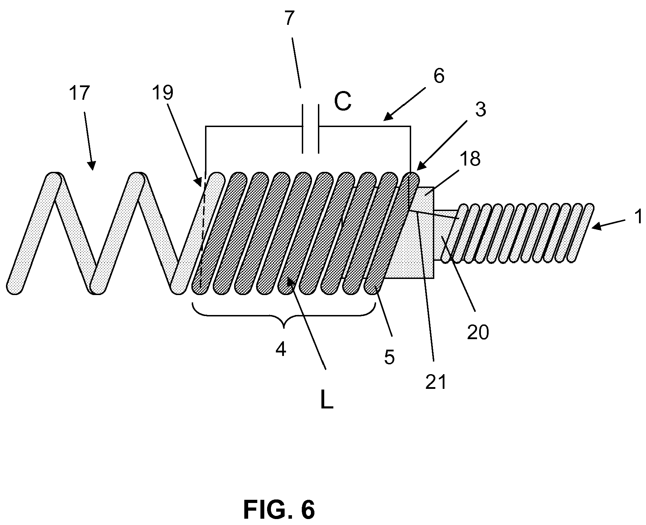

Bei der in Fig. 6 gezeigten Ausführungsform einer Elektrodenvorrichtung ist die frequenzabhängige Übertragungseinrichtung 3 in eine sogenannte Fixierhelix 17 integriert, die das distale Ende einer beispielsweise als Schrittmacherelektrode ausgebildeten Elektrodenvorrichtung darstellt. Wie aus der Zeichnung hervorgeht, geht die Zuleitung 1 in einen Gehäuseansatz 18 über, auf dem die proximalseitigen Windungen der Fixierhelix 17 befestigt sind. Der sich an die Befestigung anschließende Abschnitt 4 ist analog Fig. 1 wiederum als Induktivität L mit einer gut leitenden Beschichtung 5 der Wendelwindungen ausgestaltet. Der schematisch angedeutete Kondensator 7 mit seiner Kapazität C kann im Innenlumen 19 des Abschnittes 4 der Fixierhelix 17 beispielsweise in dem Gehäuseansatz 18 untergebracht sein. Sein Gehäuse bildet damit die Schulter 20, an der die Zuleitung 1 befestigt ist. Zwischen dieser und dem Abschnitt 4 ist eine elektrische Verbindung 21 vorgesehen. Das distale Ende der Fixierhelix 17 ist mit axial aufgeweiteten Windungen versehen, die durch eine Rotation der Fixierhelix um ihre Längsachse im Körpergewebe "eingeschraubt" werden können. Der die Induktivität L bildende Abschnitt 4 der Fixierhelix 17 ist im Übrigen in den Bereichen, wo er in Gewebekontakt gelangen kann, nach außen hin isoliert. Die Kontaktierung der Zuleitung 1 und die elektrische Verbindung 21 im Bereich der Schulter erfolgt durch ein Vercrimpen.

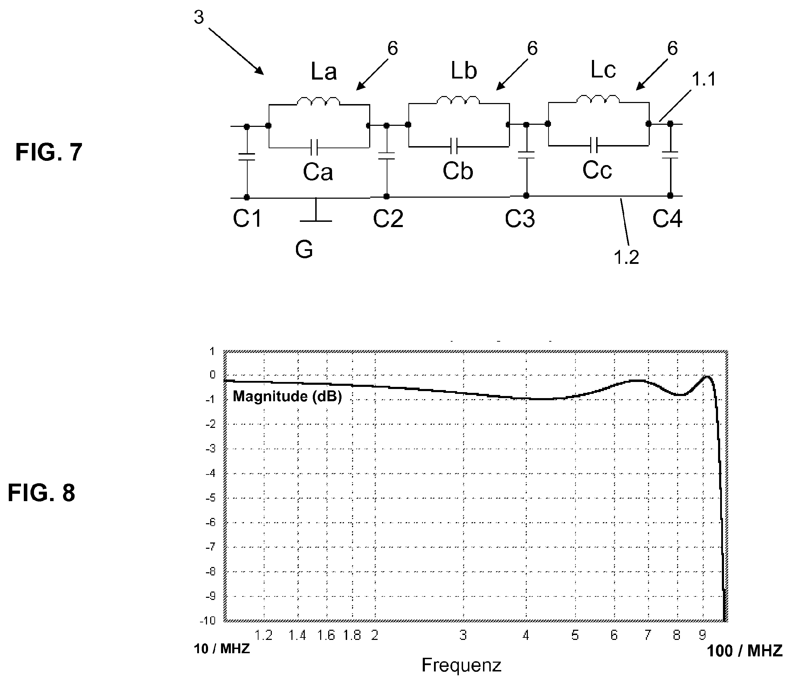

In Fig. 7 ist eine erste schaltungstechnische Realisierung der frequenzabhängigen Übertragungseinrichtung 3 bei zwei Zuleitungen 1.1, 1.2 dargestellt, die Widerstände, Induktivitäten, Kapazitäten und Übertrager einsetzt. Umgesetzt ist ein elliptischer Tiefpass höherer Ordnung, wobei seriell hintereinander geschaltet Parallelkreise von Induktivitäten La, Lb, Lc und Kapazitäten Ca, Cb, Cc in die Zuleitung 1.1 eingeschaltet sind. Eine Ankopplung an die Zuleitung 1.2 erfolgt über Kapazitäten C1, C2, C3, C4 endseitig und zwischen den LC-Kreisen. Die Zuleitung 1.1. versorgt dabei einen Kontaktpol der Elektrodenvorrichtung, während die zweite Zuleitung 1.2 zu einem weiteren Kontaktpol, wie beispielsweise einem Ring-Elektrodenpol oder einer ICD-Schockwendel führt. Falls kein weiterer solcher Pol vorhanden ist, kann die Zuleitung 1.2 auch zu einem Potentialreferenzpol führen, der nur zu diesem Zweck als Kontakt zum Gewebe realisiert ist, der sonst jedoch keine weitere Funktion für Diagnose oder Therapie hat. In Fig. 7 ist der Potentialreferenzpol mit G (Ground) bezeichnet.

Fig. 8 zeigt beispielhaft den Frequenzgang einer frequenzabhängigen Übertragungseinrichtung zwischen 10 und 100 MHz. Erkennbar erfolgt bis kurz vor der Grenzfrequenz von 100 MHz keine relevante Dämpfung, die dann mit einer scharfen Flanke auf eine Dämpfung von > 10 dB ansteigt.

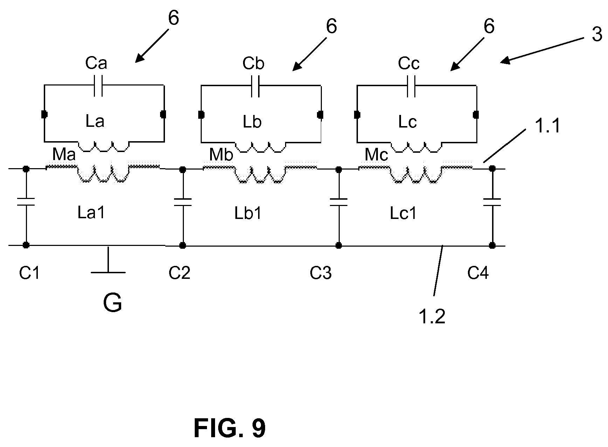

Fig. 9 zeigt eine frequenzabhängige Übertragungseinrichtung 3 mit Schwingkreisen 6 in Form von LC-Resonatoren, die mit ihren Induktivitäten La, Lb, Lc über Einkoppelinduktivitäten La1, Lb1, Lc1 in der Zuleitung 1.1 induktiv eingekoppelt sind. Im Übrigen entspricht die Ausführung gemäß Fig. 9 der von Fig. 7, so dass auf die dortige Beschreibung verwiesen werden kann.

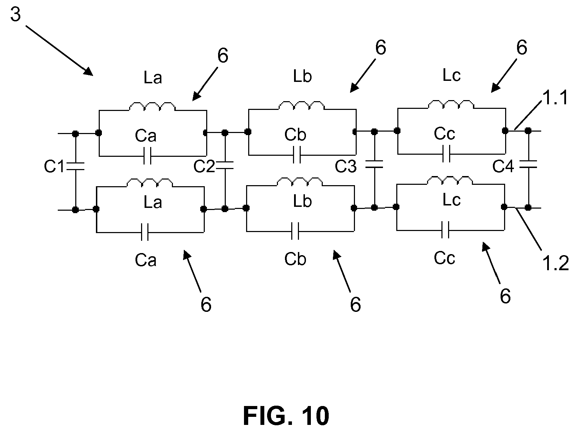

In Fig. 10 ist eine frequenzabhängige Übertragungseinrichtung 3 gezeigt, die in zwei Zuleitungen 1.1, 1.2 zu nicht näher dargestellten Kontaktpolen realisiert ist. In jeder Zuleitung 1.1, 1.2 sind drei Schwingkreise 6 jeweils mit Induktivität La, Lb, Lc und parallel dazu geschalteter Kapazität Ca, Cb, Cc in Reihe geschaltet. Die beiden Zuleitungen 1.1, 1.2 sind ferner über Kapazitäten C1, C2, C3, C4 endseitig und zwischen den Schwingkreisen kapazitiv gekoppelt. In dieser Ausführung sind symmetrisch balancierte Tiefpassfilter-Eigenschaften erreicht.

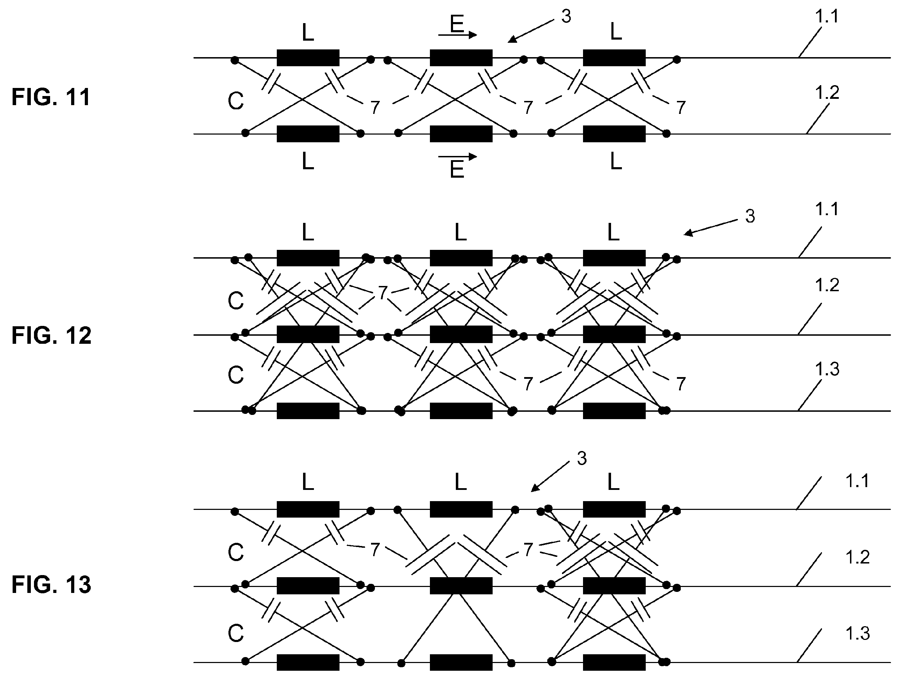

In Fig. 11 ist eine frequenzabhängige Übertragungseinrichtung 3 für zwei Zuleitungen 1.1, 1.2 gezeigt, bei denen jeweils seriell in die Zuleitungen 1.1, 1.2 Induktivitäten L eingeschaltet sind. Die Zuleitungen 1.1, 1.2 sind sodann kreuzweise bezogen auf die jeweiligen Induktivitäten L kapazitiv durch die Kondensatoren 7 mit der Kapazität C verkoppelt. Zweck dieser Verschaltung ist die weitestgehende Auslöschung von in den Induktivitäten durch elektromagnetisches Wechselfeld verursachte Spannungen. Dies beruht darauf, dass die beiden Zuleitungen 1.1, 1.2 eine im Wesentlichen gleiche, tangentiale E-Feldstärke erfahren, da sie geometrisch nahe benachbart liegen. Die in den Induktivitäten L verursachten Spannungen überlagern sich aufgrund der verkreuzten Ankopplung durch die Kondensatoren 7 gegenphasig, wodurch eine Auslöschung erfolgt.

Eine analoge Kreuzkopplung von Induktivitäten in drei Zuleiten 1.1, 1.2, 1.3 zeigen die Fig. 12 und 13. Während in Fig. 12 alle Kreuzkombinationen durch Kondensatoren 7 gekoppelt sind, sind bei der Ausführungsform gemäß Fig. 13 einige Koppelkondensatoren weggelassen, was zu keiner signifikanten Beeinträchtigung der Spannungsauslöschung führt.

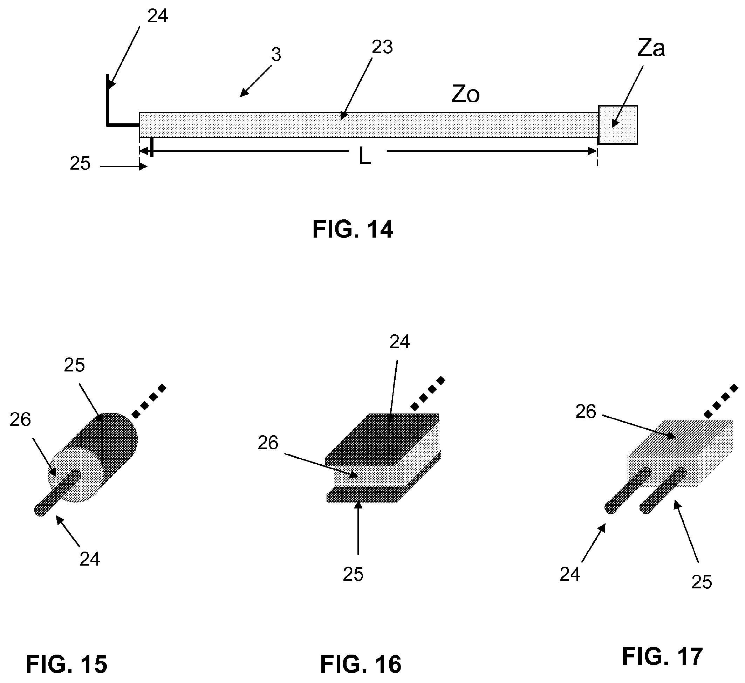

In den Fig. 14 bis 22 sind frequenzabhängige Übertragungseinrichtungen 3 gezeigt, die auf dem Prinzip von Wellenleitern beruhen. Wie aus den Fig. 15 bis 17 deutlich wird, bestehen diese Wellenleiter 23 aus zwei benachbarten Leitern 24, 25, die über ein Dielektrikum 26 gekoppelt sind. Der Wellenleiter 23 ist damit durch eine spezifische Wellenimpedanz Z0 charakterisiert. In Abhängigkeit der Abschlussimpedanz Za, die die beiden Leiter 24, 25 des Wellenleiters 23 verbindet, stellt sich dann am entgegengesetzten Ende zwischen diesen Leitern 24, 25 eine Impedanz Z ein. Die Abschlussimpedanz Za kann beispielsweise durch eine Schaltung von Widerständen R, Induktivitäten L, Kapazitäten C und Überträgern Ü realisiert sein. Die Bauelemente sind so zu dimensionieren, dass bei einer gegebenen Länge l des Wellenleiters 23 sich die gewünschte Impedanz Z am anderen Ende einstellt. Um eine Filterung bei einer bestimmten Frequenz, also beispielsweise eine Sperre zu realisieren, müssen Za und l so ausgelegt sein, dass Z im Idealfall eine durchtrennte Leitung simuliert. Dann liegt der Fall einer frequenzabhängigen Leitungsunterbrechung vor. Im einfachsten, bevorzugten Fall ist Za ein Kurzschluss, dann können die niederfrequenten Therapie- und Diagnoseströme ungestört fließen. Dazu wird in Abhängigkeit der spezifischen Wellenimpedanz Z0 die Länge entsprechend bestimmt, wozu eine sogenannte "Smith-Chart" verwendet wird.

Um zwischen zwei Kontaktpolen bei einer bestimmten Frequenz eine gute Leitfähigkeit zu realisieren, um beispielsweise unerwünschte Störströme umzuverteilen, wird verglichen zu den vorstehenden Ausführungen genau umgekehrt vorgegangen. Der Abschluss Za bleibt offen, über die Länge l des Wellenleiters 23 wird die Impedanz dann so transformiert, dass bei der entsprechenden Frequenz am anderen Ende ein frequenzabhängiger Kurzschluss entsteht. Für niederfrequente Ströme besteht dieser Kurzschluss nicht, so dass Therapie und Diagnose nicht beeinträchtigt sind.

Gemäß Fig. 15 ist der Wellenleiter zylindrisch ausgeführt, wobei ein koaxialer Innenleiter 24 und ein rohrförmiger Außenleiter 25 durch das Dielektrikum 26 gekoppelt sind.

Bei der Ausführungsform gemäß Fig. 16 sind planare, langgestreckte Leiter 24, 25 sandwichartig angeordnet und dazwischen das Dielektrikum 26 gesetzt.

Gemäß Fig. 17 werden zwei drahtförmige Leiter 24, 25 in einem stangenförmigen Block des Dielektrikums 26 positioniert.

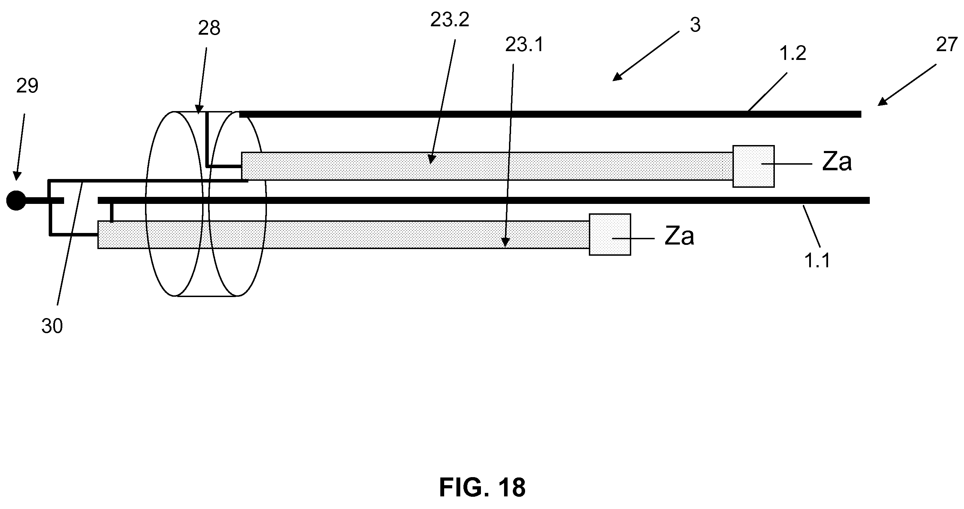

Eine schaltungstechnische Realisierung einer frequenzabhängigen Übertragungseinrichtung in einer Elektrodenvorrichtung mit zwei Zuleitungen 1.1, 1.2 unter Verwendung von Wellenleitern 23.1, 23.2 ist in Fig. 18 dargestellt. Schematisch ist das distale Ende einer Elektrodenvorrichtung in Form einer Herzelektrode 27 dargestellt, die einen Ring-Kontaktpol 28 und einen Spitzenpol (Tip-Elektrode) 29 aufweist. In die Zuleitung 1.1 ist der erste Wellenleiter 23.1 eingeschaltet, die Zuleitung 1.2 versorgt den Ring-Kontaktpol 28. In einer Leitungsverbindung 30 zwischen Ring-Kontaktpol 28 und Spitzen-Kontaktpol 29 ist der zweite Wellenleiter 23.2 eingeschaltet.

Mit Hilfe des Wellenleiters 23.1 wird auf der Zuleitung 1.1 eine frequenzabhängige Leitungsunterbrechung (Stromsperre) realisiert. Zwischen den Kontaktpolen 28, 29 wird mit Hilfe des Wellenleiters 23.2 ein frequenzabhängiger Kurzschluss hervorgerufen. Die Zuleitung 1.2 kann im Übrigen auch einen Wellenleiter eingebaut haben, wie dies bei der Zuleitung 1.1 der Fall ist. Dann wird kurz vor dem Ring-Kontaktpol 28 eine Unterbrechungsstelle realisiert.

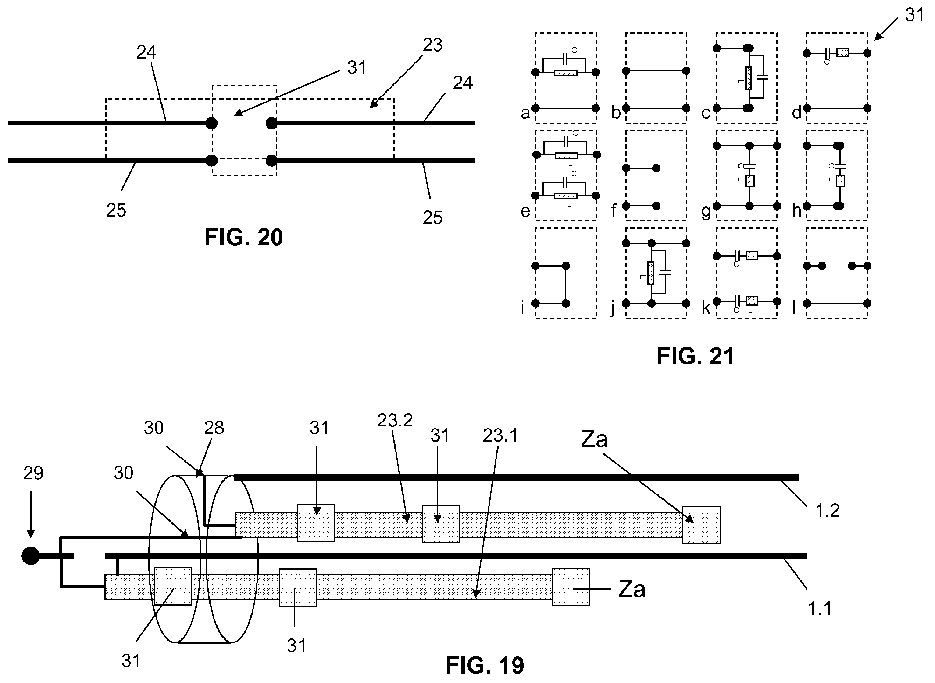

Die in Fig. 19 dargestellte Ausführungsform einer Elektrodenvorrichtung 27 mit zwei Zuleitungen 1.1 und 1.2, Ring-Kontaktpol 28 und Spitzen-Kontaktpol 29 entspricht dem Ausführungsbeispiel gemäß Fig. 18. Gleichermaßen sind zwei Wellenleiter 23.1, 23.2 in übereinstimmender Weise in die Zuleitung 1.1 bzw. Leitungsverbindung 30 eingekoppelt.

Der wesentliche Unterschied besteht darin, dass durch in die Wellenleiter 23 integrierte Netzwerke 31 die gebildete frequenzabhängige Übertragungseinrichtung 3 gleichzeitig für den Betrieb bei mehreren Frequenzen einsetzbar ist. Die in die Wellenleiter 23 zwischengeschalteten Netzwerke 31 sind jeweils für eine bestimmte MRT-Frequenz aktiv, so dass ein Patient mit einer wie in Fig. 19 ausgelegten implantierten Herzelektrode bei gängigen MRI-Systemen mit 1,5 T, 3 T und 7 T Magnetfeldstärke ohne Probleme bleiben.

Fig. 20 in Verbindung mit Fig. 21 visualisiert die Zwischenschaltung der Netzwerke 31 in die Leiter 24, 25 der Wellenleiter 23. So sind die Leiter 24, 25 unterbrochen, an ihren Anschlussstellen 32 sind die in Fig. 21a bis l dargestellten Netzwerkrealisierungen anschließbar. Wie sich aus den Einzeldarstellungen a bis l ergibt, können verschiedene Kurzschluss- und Unterbrechungsvarianten (Fig. 21b, f, i und l), verschiedene Kombinationen von zwischengeschalteten parallelen LC-Schwingkreisen (Fig. 21, a, c, e und j) sowie von seriellen LC-Gliedern (Fig. 21, d, g, h und k) mit unterschiedlichen Einflüssen auf die Frequenzabhängigkeit zwischen gesetzt werden.

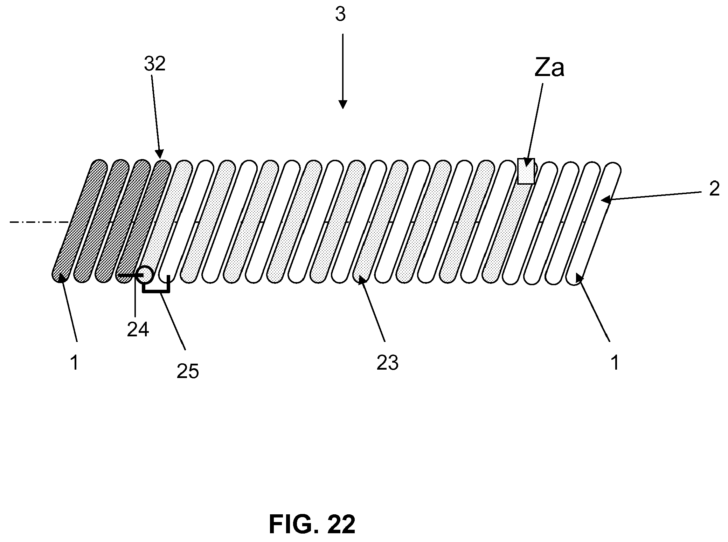

Fig. 22 zeigt eine Wellenleiter-Variante der frequenzabhängigen Übertragungseinrichtung 3, bei der über eine gewisse Länge der Zuleitung 1 ein gewendelter Wellenleiter 23 mit gleichem Durchmesser und gleicher Wendelsteigung eingesetzt ist. In diesem Bereich ist das "Wendelgewinde" quasi zweigängig und abwechselnd durch die Wendeln der Zuleitung 1 und des Wellenleiters 23 gebildet. Am distalen Ende 32 des Wellenleiters 23 sind dessen Leiter 24, 25 mit dem hin- bzw. wegführenden Abschnitt der Zuleitung 1 verbunden. In Fig. 22 ist schließlich noch der Wellenleiterabschluss Za am proximalen Ende des Wellenleiters 23 dargestellt.

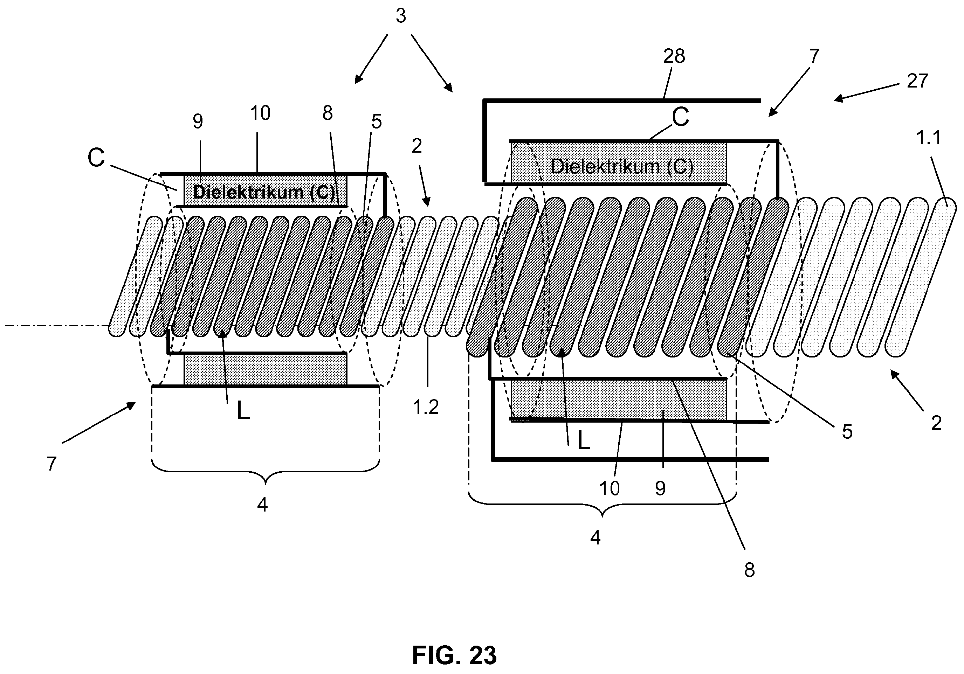

Die Fig. 23 bis 26 zeigen komplexere Implementierungen von Elektrodenvorrichtungen 27 mit frequenzabhängigen Übertragungseinrichtungen 3. So zeigt Fig. 23 eine Herzelektrode 27 mit einem gewendelten Außenleiter als Zuleitung 1.1 zu einem Ring-Kontaktpol 28. In diesem Außenleiter läuft ein Innenleiter als Zuleitung 1.2 über das distale Ende der Zuleitung 1.1 hinaus und schließt beispielsweise eine (nicht gezeigte) Tip-Elektrode elektrisch an.

Die äußere Zuleitung 1.1 ist an ihrem Ende über einen Abschnitt 4 als Induktivität L durch eine entsprechend hochleitfähige Beschichtung 5 ausgelegt. Um diesen Abschnitt 4 herum ist analog Fig. 3 ein Zylinder-Kondensator 7 mit Innenhülse 8, Dielektrikum 9 und Außenhülse 10 positioniert, der innerhalb des Ring-Kontaktpols 28 liegt. Letzterer ist mit der Innenhülse 8 des Kondensators 7 elektrisch verbunden, die wiederum am distalen Ende der Induktivität L angeschlossen ist. Deren proximales Ende ist mit der Außenhülse 10 elektrisch verbunden, so dass zwischen Zuleitung 1.1 und Ring-Kontaktpol 28 eine Parallelschaltung der Induktivität L und der Kapazität C des Kondensators 7 liegt.

Eine analoge frequenzabhängige Übertragungseinrichtung 3 ist im Bereich der inneren Zuleitung 1.2 distal zu der Übertragungseinrichtung 3 im Außenleiter versetzt angeordnet. Auch hier ist wiederum eine Induktivität L in der beschriebenen Weise innerhalb der gewendelten Zuleitung 1.2 angelegt, die an ihren abgewandten endseitigen Wendeln mit der Außen- bzw. Innenhülse des Zylinderkondensators 7 elektrisch verbunden ist.

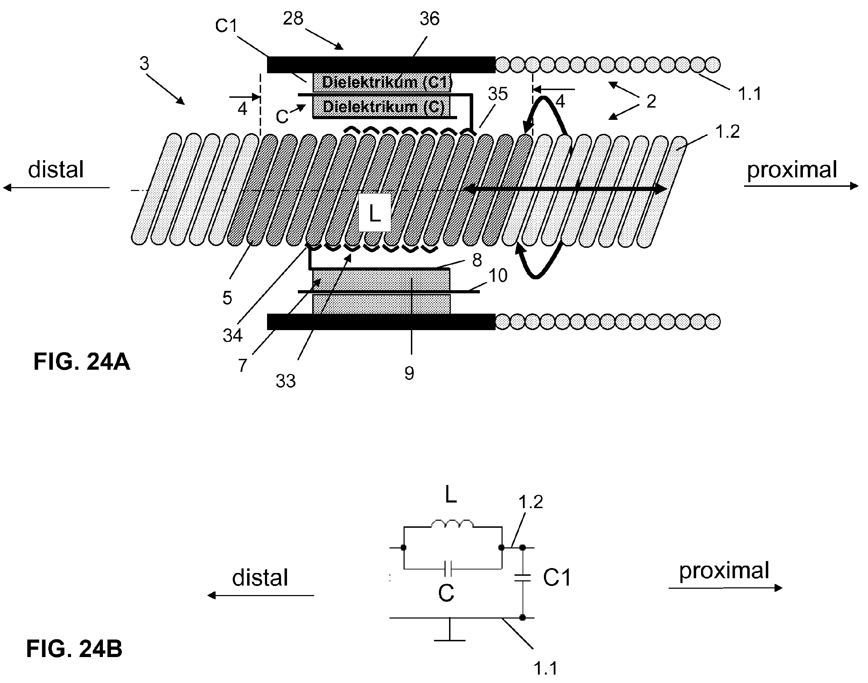

Die in Fig. 24A und B gezeigte Ausführungsform gibt die Realisierung einer frequenzabhängigen Übertragungseinrichtung 3 in Form einer frequenzabhängigen Stromsperre entlang der inneren Zuleitung 1.2 einer Herzelektrode mit Hochpassverhalten zwischen der äußeren Zuleitung 1.1 und der inneren Zuleitung 1.2 wieder. Dabei ist für die gewendelte innere Zuleitung 1.2 ein sogenannter Steigungsgeber 33 in die Übertragungseinrichtung 3 integriert. Dieser Steigungsgeber 33 ist in Form eines Innengewindes ausgelegt, in dem der als Induktivität L in der beschriebenen Weise ausgebildete Abschnitt 4 der inneren Zuleitung 1.2 drehbar und damit bei einer Rotation in axialer Richtung verschiebbar gelagert ist. Die jeweils in distaler bzw. proximaler Richtung letzten Gewindegänge 34, 35 des Steigungsgebers 33 sind jeweils elektrisch leitfähig ausgebildet und kontaktieren mit der darin geführten Windung der die Induktivität L bildenden Wendel. Der distal gelegene Gewindegang 34 ist dabei mit der Innenhülse 8 eines um die Induktivität L herum angeordneten Zylinderkondensators 7 elektrisch verbunden, der proximale Gewindegang 35 mit der entsprechenden Außenhülse 10. Der durch das Dielektrikum 9 zwischen den beiden Hülsen 8, 10 gebildete Kondensator C ist damit parallel zu der Induktivität L zwischen den beiden Gewindegängen 34, 35 in der Zuleitung 1.2 zum Spitzen-Kontaktpol 29 geschaltet, wie dies in dem Ersatzschaltbild gemäß Fig. 24B deutlich wird. Der Ring-Kontaktpol 28, der über ein weiteres Dielektrikum 36 an die Außenhülse 10 des Kondensators 7 angebunden ist, bildet eine kapazitive Kopplung C1 zwischen der äußeren Zuleitung 1.1 bzw. dem Ring-Kontaktpol 28 und der inneren Zuleitung 1.2.

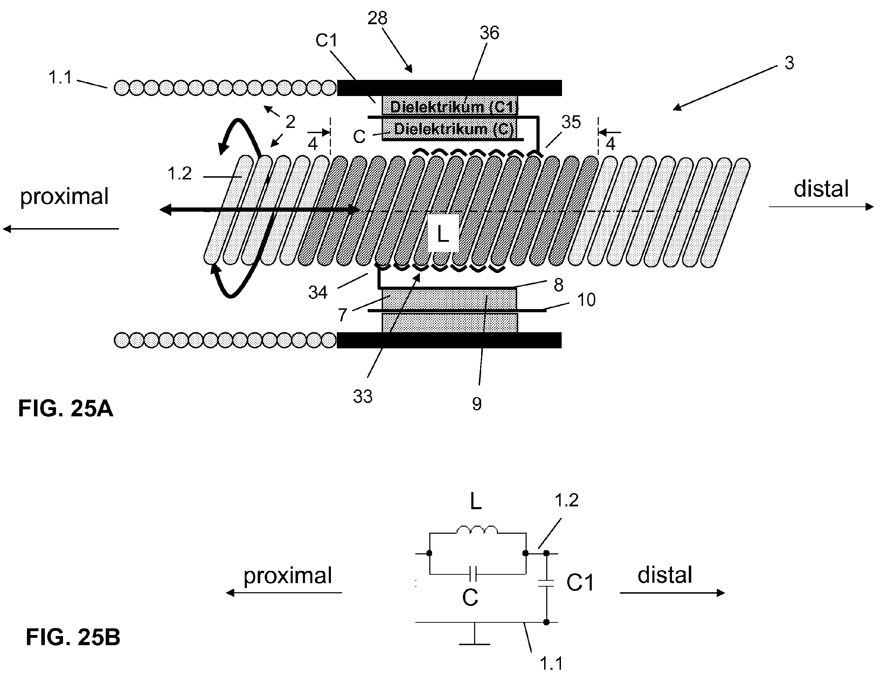

Die in Fig. 25 dargestellte Ausführungsform unterscheidet sich von der Fig. 24 nur dadurch, dass die von der Elektrodenleitung aufgenommene Energie auf unterschiedliche elektronische Bauelemente trifft. Die Energie kommt im betrachten Fall vom proximalen Ende und trifft in der Fig. 25 zunächst auf einen Spannungsteiler, während in Fig. 24 die Energie zunächst auf den Ableitkondensator trifft und restliche Energie wird durch den seriell liegenden Schwingkeis (Sperrkreis) gedämpft.

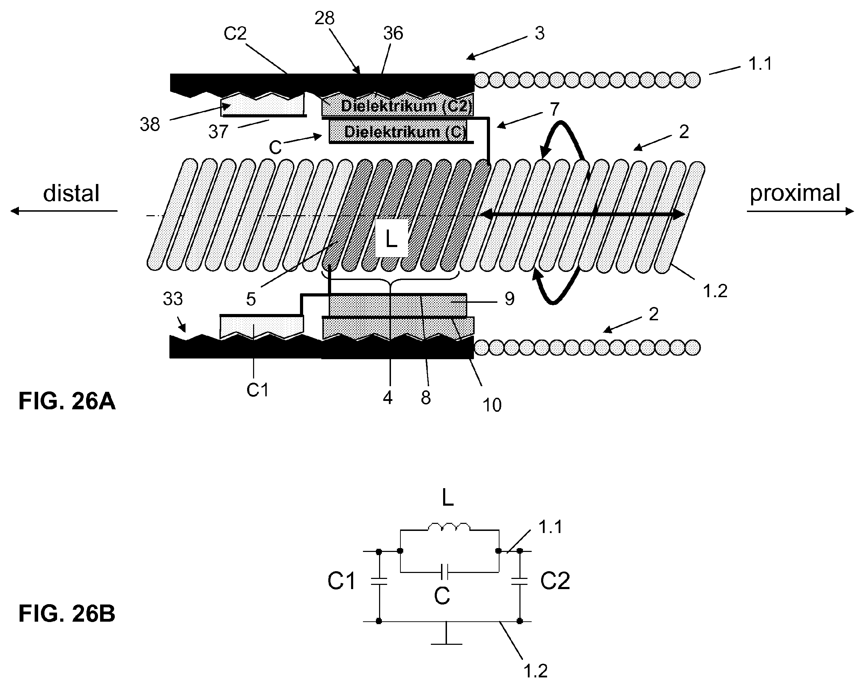

Fig. 26 zeigt ausschnittsweise eine Elektrodenvorrichtung, bei dem für die gewendelte innere Zuleitung 1.2 mit einem integrierten Steigungsgeber 33 ein Tiefpass für die innere Zuleitung 1.2 realisiert ist. Im Einzelnen ist wieder über einen Abschnitt 4 eine Induktivität L durch eine hochleitfähige Beschichtung oder dergleichen in der gewendelten Zuleitung 1.2 angelegt. Die jeweils in proximaler bzw. distaler Richtung letzten Wendelgänge der Induktivität L sind zum einen mit der Innenhülse 8 bzw. Außenhülse 9 eines Zylinderkondensators 7, der analog Fig. 3 aufgebaut ist, elektrisch angebunden, so dass wiederum eine Parallelschaltung der Glieder L und C erhalten wird (s. Fig. 26 b).

Um diese Anordnung herum ist ein Ring-Kontaktpol 28 platziert, der über die äußere gewendelte Zuleitung 1.1 an das Implantat angeschlossen ist. An der Innenseite des Ring-Kontaktpols 28 ist ein Steigungsgeber 33 in Form von Gewindegängen ausgebildet, der zum einen über ein Dielektrikum 36 an die Außenhülse 10 des Zylinderkondensators 7 kapazitiv angekoppelt ist. Damit wird die Kapazität C2 zwischen den Zuleitungen 1.1 und 1.2 realisiert, wie dies in Fig. 26b erkennbar ist.

Ferner ist eine elektrisch mit der Innenhülse des Zylinderkondensators 7 verbundene Ringhülse 37 distal vor dem Zylinderkondensator 7 innerhalb des Ring-Kontaktpols 28 angeordnet, die wiederum über ein Dielektrikum 38 an den Ring-Kontaktpol 28 kapazitiv mit der Kapazität C1 angekoppelt ist. Damit sind die beiden Zuleitungen 1.1, 1.2 auch distal des LC-Schwingkreises kapazitiv über den Kondensator C1 gekoppelt (s. Fig. 26 b).

**Bezugszeichenliste**

| | | | |
|---|---|---|---|
| 1 | Zuleitung ab Fig. 7: 1.1, 1.2 | 31 | Netzwerk |
| 2 | Wendel | 32 | distales Ende |
| 3 | Einrichtung | 33 | Steigungsgeber |
| 4 | Abschnitt | 34 | Gewindegang - distal |
| 5 | Beschichtung | 35 | Gewindegang - proximal |
| 6 | Schwingkreis | 36 | Dielektrikum |
| 7 | Kondensator | 37 | Ringhülse |
| 8 | Innenhülse | 38 | Dielektrikum |
| 9 | Dielektrikum | | |
| 10 | Außenhülse | l | Länge von Wellenleiter |
| 11 | Trimmelement | L | Induktivität |
| 12 | Sensor | C | Kapazität |
| 13 | f/U-Wandler | Z1 | Impedanz |
| 14 | Demodulator | Z2 | Impedanz |
| 15 | Schwellwertgeber | G | Ground |
| 16 | Tiefpass | Za | Abschlussimpedanz |
| 17 | Fixierhelix | | |
| 18 | Gehäuseansatz | | |
| 19 | Innenlumen | | |
| 20 | Schulter | | |
| 21 | elektrische Verbindung | | |
| 22 | aufgeweitete Windungen | | |
| 23 | Wellenleiter | | |
| 24 | Leiter | | |
| 25 | Leiter | | |
| 26 | Dielektrikum | | |
| 27 | Herzelektrode | | |
| 28 | Ringkontaktpol | | |
| 29 | Spitzenkontaktpol | | |
| 30 | Leitungsverbindung | | |

## Patentansprüche

1. Elektrodenvorrichtung zur Strom- oder Spannungsführung zwischen einem implantierbaren elektromedizinischen Gerät und einem Therapie- und/oder Diagnoseort im menschlichen Körper, umfassend mindestens eine strom-/spannungsführende Zuleitung (1) und mindestens einen elektrischen Kontaktpol zu einem Körperteil, wobei der Zuleitung (1) mindestens eine Einrichtung (3) mit einem frequenzabhängigen Übertragungsverhalten zugeordnet ist, wobei diese mindestens eine frequenzabhängige Übertragungseinrichtung (3) durch einen oder mehrere Abschnitte (4) der strom-/spannungsführenden Zuleitung (1) selbst gebildet ist und wobei diese mindestens eine frequenzabhängige Übertragungseinrichtung (3) therapeutisch und/oder diagnostisch unerwünschte Signale in mindestens einem definierten Frequenzbereich zumindest teilweise ausfiltert **dadurch gekennzeichnet, dass** die frequenzabhängige Übertragungseinrichtung (3) durch einen höher leitfähigen Abschnitt (4) einer Wendel (2) der strom-/spannungsführenden Zuleitung gebildet ist.

2. Elektrodenvorrichtung nach Anspruch 1, **dadurch gekennzeichnet, dass** das frequenzabhängige Übertragungsverhalten einem Tiefpass-, Bandsperr- oder Hochpass-Filter entspricht.

3. Elektrodenvorrichtung nach Anspruch 1 oder 2, **dadurch gekennzeichnet, dass** die frequenzabhängige Übertragungseinrichtung (3) eine Durchführung mit einem vorzugsweisen Mindestdurchmesser von 0,2 mm für einen Führungsdraht, Guidewire, Mandrin oder dergleichen aufweist.

4. Elektrodenvorrichtung nach einem der Ansprüche 1 bis 3, **dadurch gekennzeichnet, dass** der vorzugsweise eine gegenüber der verbleibenden strom-/spannungsführenden Leitung (1) mindestens doppelte Leitfähigkeit aufweisende, höher leitfähige Abschnitt (4) durch eine hoch leitfähige Beschichtung (5) oder Dotierung des Abschnittes (4) der Wendel (2) gebildet ist.

5. Elektrodenvorrichtung nach einer der Ansprüche 1 bis 4, **dadurch gekennzeichnet, dass** der höher leitfähige Abschnitt (4) der Wendel (2) mit außen isolierten Windungen versehen ist.

6. Elektrodenvorrichtung nach einem der Ansprüche 1 bis 5, **dadurch gekennzeichnet, dass** der höher leitfähige Abschnitt (4) der Wendel (2) mit einem parallel geschalteten Kondensator (7) einen Induktivität-Kapazität-Schwingkreis (6) als frequenzabhängige Übertragungseinrichtung (3) bildet oder der höher leitfähige Abschnitt (4) der Wendel (2) mit einem parallel geschalteten Kondensator (7) einen Induktivität-Kapazität-Schwingkreis (6) als frequenzabhängige Übertragungseinrichtung (3) bildet und der Kondensator des Induktivität-Kapazität-Schwingkreises (6) durch einen um den leitfähigen Abschnitt (4) der Wendel (2) herum und/oder innerhalb des leitfähigen Abschnitts (4) der Wendel (2) angeordneten Zylinder-Kondensator (7) gebildet ist.

7. Elektrodenvorrichtung nach einem der vorgenannten Ansprüche, **dadurch gekennzeichnet, dass** die Frequenzabhängigkeit der frequenzabhängigen Übertragungseinrichtung (3) variabel steuerbar ist.

8. Elektrodenvorrichtung nach Anspruch 7, **dadurch gekennzeichnet, dass** die Steuerung der Frequenzabhängigkeit mithilfe eines Trimmelementes (11), vorzugsweise ein spannungsgesteuerter Kondensator, oder durch eine Steuerspannung erfolgt.

9. Elektrodenvorrichtung nach Anspruch 8, **dadurch gekennzeichnet, dass** die Steuerspannung durch eine Programmierung des elektromedizinischen Gerätes oder durch einen Sensor (12) zur Erfassung therapeutisch und/oder diagnostisch unerwünschter Signale erzeugbar ist.

10. Elektrodenvorrichtung nach Anspruch 9, **dadurch gekennzeichnet, dass** der Sensor (12) im implantierten elektromedizinischen Gerät angeordnet oder der Zuleitung (1) zugeordnet ist.

11. Elektrodenvorrichtung nach Anspruch10, **dadurch gekennzeichnet, dass** der Sensor ein Feldsensor (12) für elektrische, magnetische und/oder elektromagnetische Felder ist, insbesondere ein Dipol ist, wobei die Felder statische und/oder Wechselfelder sind.

12. Elektrodenvorrichtung nach einem der vorgenannten Ansprüche, **dadurch gekennzeichnet, dass** die frequenzabhängige Übertragungseinrichtung (3) durch einen oder mehrere Schwingkreise (LC) gebildet ist, wobei jeder Schwingkreis (LC) induktiv angekoppelt ist.

13. Elektrodenvorrichtung nach einem der vorgenannten Ansprüche, **dadurch gekennzeichnet, dass** die frequenzabhängige Übertragungseinrichtung (3) an einer Fixierhelix (17) an der Elektrodenvorrichtung ausgebildet ist.

14. Elektrodenvorrichtung nach einem der vorgenannten Ansprüche, **dadurch gekennzeichnet, dass** die frequenzabhängige Übertragungseinrichtung (3) durch mindestens einen Wellenleiter (23) mit einer frequenzspezifischen Wellenimpedanz gebildet ist.

15. Elektrodenvorrichtung mindestens nach Anspruch 14, **dadurch gekennzeichnet, dass** der mindestens eine Wellenleiter (23) durch eine Abschlussimpedanz (Za) abgeschlossen ist und/oder zur Frequenzanpassung an mindestens zwei unerwünschte Signalfrequenzen in den mindestens einen Wellenleiter (23) mindestens zwei Netzwerke (31) zwischengeschaltet ist und/oder ein Netzwerk (31) den Wellenleiter (23) abschließt und/oder der Wellenleiter (23) durch zwei über ein Dielektrikum (26) gekoppelte Leiter (24, 25) gebildet ist und/oder der Wellenleiter (23) gewendelt und in eine als Wendelleitung ausgebildete strom-/spannungsführende Zuleitung (1) integriert ist.

## Claims

1. An electrode unit for carrying current or voltage between an implantable electromedical device and a site for therapy and/or diagnosis in the human body, comprising at least one current/voltage-carrying lead (1) and at least one electric contact pole to a body part, wherein the lead (1) is assigned at least one device (3) having frequency-dependent transmission behavior, wherein this at least one frequency-dependent transmission device (3) is formed by one or multiple sections (4) of the current/voltage-carrying lead (1) itself, and wherein this at least one frequency-dependent transmission device (3) at least partially filters out therapeutically and/or diagnostically unwanted signals in at least one defined frequency range, **characterized in that** the frequency-dependent transmission device (3) is formed by a highly conductive section (4) of a coil (2) of the current/voltage-carrying lead.

2. The electrode unit according to claim 1, **characterized in that** the frequency-dependent transmission behavior corresponds to a low-pass filter, a band-stop filter, or a high-pass filter.

3. The electrode unit according to claim 1 or 2, **characterized in that** the frequency-dependent transmission device (3) has a passage having a preferably minimum diameter of 0.2 mm for a guide wire, a mandrel, or the like.

4. The electrode unit according to any one of claims 1 to 3, **characterized in that** the preferably highly conductive section (4) having conductivity which is at least twice that of the remaining current/voltage-carrying line (1) is formed by a highly conductive coating (5) or doping of the section (4) of the coil (2).

5. The electrode unit according to any one of claims 1 to 4, **characterized in that** the highly conductive section (4) of the coil (2) is provided with externally insulated turns.

6. The electrode unit according to any one of claims 1 to 5, **characterized in that** the highly conductive section (4) of the coil (2) having a capacitor (7) connected in parallel forms an inductance-capacitance oscillating circuit (6) as the frequency-dependent transmission device (3), or the highly conductive section (4) of the coil (2) having a capacitor (7) connected in parallel forms an inductance-capacitance oscillating circuit (6) as the frequency-dependent transmission device (3), and the capacitor of the inductance-capacitance oscillating circuit (6) is formed by a cylindrical capacitor (7) disposed around the conductive section (4) of the coil (2) and/or within the conductive section (4) of the coil (2).

7. The electrode unit according to any one of the preceding claims, **characterized in that** the frequency dependence of the frequency-dependent transmission device (3) is variably controllable.

8. The electrode unit according to claim 7, **characterized in that** the control of the frequency dependence is carried out with the aid of a trimming element (11), preferably a voltage-controlled capacitor, or by a control voltage.

9. The electrode device according to claim 8, **characterized in that** the control voltage can be generated by means of a programming of the electromedical device or by means of a sensor (12) for sensing therapeutically and/or diagnostically unwanted signals.

10. The electrode device according to claim 9, **characterized in that** the sensor (12) is disposed in the implanted electromedical device or is assigned to the lead (1).

11. The electrode unit according to claim 10, **characterized in that** the sensor is a field sensor (12) for electrical, magnetic and/or electromagnetic fields, in particular is a dipole, wherein the fields are static and/or alternating fields.

12. The electrode unit according to any one of the preceding claims, **characterized in that** the frequency-dependent transmission device (3) is formed by one or multiple oscillating circuits (LC), wherein each oscillating circuit (LC) is inductively coupled.

13. The electrode unit according to any one of the preceding claims, **characterized in that** the frequency-dependent transmission device (3) is formed on a fixing helix (17) on the electrode unit.

14. The electrode unit according to any one of the preceding claims, **characterized in that** the frequency-dependent transmission device (3) is formed by at least one waveguide (23) having frequency-dependent wave impedance.

15. The electrode unit according to at least claim 14, **characterized in that** the at least one waveguide (23) is terminated by a terminal impedance (Za) and/or at least two networks (31) are inserted into the at least one waveguide (23) for the purpose of frequency adaptation to at least two unwanted signal frequencies, and/or a network (31) terminates the waveguide (23), and/or the waveguide (23) is formed by two conductors (24, 25) coupled via a dielectric (26), and/or the waveguide (23) is coiled and is integrated into a current/voltage-carrying supply lead (1) as a helical line.

## Revendications

1. Dispositif d'électrodes pour la commande de courant ou de tension entre un appareil électromédical implantable et un lieu de thérapie et/ou de diagnostic dans le corps humain, comprenant au moins une ligne d'alimentation (1) conduisant le courant / la tension et au moins un pôle de contact électrique vers une partie du corps, où au moins un équipement (3) avec des caractéristiques de transmission dépendant de la fréquence est associé à la ligne d'alimentation (1), où cet au moins un équipement de transmission (3) dépendant de la fréquence est formé par un ou plusieurs segments (4) de la ligne d'alimentation (1) conduisant le courant / la tension elle-même et où cet au moins un équipement de transmission (3) dépendant de la fréquence filtre au moins partiellement les signaux thérapeutiques et/ou diagnostiques non souhaités dans au moins une zone de fréquences définie, **caractérisé en ce que** l'équipement de transmission (3) dépendant de la fréquence est formé par un segment (4) hautement conducteur d'une hélice (2) de la ligne d'alimentation conduisant le courant / la tension.

2. Dispositif d'électrodes selon la revendication 1, **caractérisé en ce que** les caractéristiques de transmission dépendant de la fréquence correspondent à celles d'un filtre passe-bas, d'un filtre coupe-bande ou d'un filtre passe-haut.

3. Dispositif d'électrodes selon les revendications 1 ou 2, **caractérisé en ce que** l'équipement de transmission (3) dépendant de la fréquence présente un passage avec un diamètre minimal de préférence de 0,2 mm pour un guide-fil, un filguide, un mandrin ou similaire.

4. Dispositif d'électrodes selon l'une des revendications 1 à 3, **caractérisé en ce que** le segment (4) hautement conducteur, présentant de préférence au moins une conductivité double par rapport à la conduite (1) conduisant le courant / la tension restante, est formé par un revêtement (5) hautement conducteur ou un dopage du segment (4) de l'hélice (2).

5. Dispositif d'électrodes selon l'une des revendications 1 à 4, **caractérisé en ce que** le segment (4) hautement conducteur de l'hélice (2) est pourvu d'enroulements isolants.

6. Dispositif d'électrodes selon l'une des revendications 1 à 5, **caractérisé en ce que** le segment (4) hautement conducteur de l'hélice (2) forme avec un condensateur (7) branché en parallèle un circuit résonnant inductance-capacité (6) servant d'équipement de transmission (3) dépendant de la fréquence, ou le segment (4) hautement conducteur de l'hélice (2) forme avec un condensateur (7) branché en parallèle un circuit résonnant inductance-capacité (6) servant d'équipement de transmission (3) dépendant de la fréquence, et le condensateur du circuit résonnant inductance-capacité (6) est formé par un condensateur cylindrique (7) disposé autour du segment (4) conducteur de l'hélice (2) et/ou à l'intérieur du segment (4) conducteur de l'hélice (2).

7. Dispositif d'électrodes selon l'une des revendications précitées, **caractérisé en ce que** la dépendance vis-à-vis de la fréquence de l'équipement de transmission (3) dépendant de la fréquence peut être commandée de manière variable.

8. Dispositif d'électrodes selon la revendication 7, **caractérisé en ce que** la commande de la dépendance de la fréquence est effectuée à l'aide d'un élément de calage (11), de préférence, un condensateur commandé en tension, ou par une tension de commande.

9. Dispositif d'électrodes selon la revendication 8, **caractérisé en ce que** la tension de commande peut être générée par une programmation de l'appareil électromédical ou par un capteur (12) pour la détermination de signaux thérapeutiques et/ou diagnostiques non souhaités.

10. Dispositif d'électrodes selon la revendication 9, **caractérisé en ce que** le capteur (12) est disposé dans l'appareil électromédical implanté ou est adjoint à la ligne d'alimentation (1).

11. Dispositif d'électrodes selon la revendication 10, **caractérisé en ce que** le capteur est un capteur de champ (12) pour des champs électriques, magnétiques et/ou électromagnétiques, est notamment un dipôle, où les champs sont des champs statiques et/ou alternatifs.

12. Dispositif d'électrodes selon l'une des revendications précitées, **caractérisé en ce que** l'équipement de transmission (3) dépendant de la fréquence est formé par un ou plusieurs circuits oscillants (LC), où chaque circuit oscillant (LC) est couplé de manière inductive.

13. Dispositif d'électrodes selon l'une des revendications précitées, **caractérisé en ce que** l'équipement de transmission (3) dépendant de la fréquence est formé sur une hélice de fixation (17) sur le dispositif d'électrodes.

14. Dispositif d'électrodes selon l'une des revendications précitées, **caractérisé en ce que** l'équipement de transmission (3) dépendant de la fréquence est formé par au moins un guide d'ondes (23) avec une impédance d'onde spécifique à la fréquence.

15. Dispositif d'électrodes au moins selon la revendication 14, **caractérisé en ce que** l'au moins un guide d'ondes (23) est terminé par une impédance de terminaison (Za) et/ou, pour l'adaptation de la fréquence à au moins deux fréquences de signal non souhaitées dans l'au moins un guide d'ondes (23), est branché entre au moins deux réseaux (31), et/ou un réseau (31) termine le guide d'ondes (23), et/ou le guide d'ondes (23) est formé par deux conducteurs (24, 25) couplés par l'intermédiaire d'un diélectrique (26), et/ou le guide d'ondes (23) est en hélice et est intégré dans une ligne d'alimentation (1) conduisant le courant / la tension conçue sous forme d'une conduite hélicoïdale.
